(19) **Europäisches Patentamt**
**European Patent Office**
**Office européen des brevets**

(11) **EP 3 314 213 B1**

(12) **EUROPEAN PATENT SPECIFICATION**

(45) Date of publication and mention
of the grant of the patent:
**06.05.2020 Bulletin 2020/19**

(21) Application number: **16732701.4**

(22) Date of filing: **21.06.2016**

(51) Int Cl.:
*A61B 5/087* (2006.01)   *A61M 16/10* (2006.01)
*G01N 21/03* (2006.01)   *G01N 21/3504* (2014.01)
*G01N 21/39* (2006.01)   *G01F 1/36* (2006.01)
*A61B 5/083* (2006.01)   *A61M 16/00* (2006.01)

(86) International application number:
**PCT/GB2016/051859**

(87) International publication number:
**WO 2017/001824 (05.01.2017 Gazette 2017/01)**

(54) **IMPROVEMENTS IN OR RELATING TO GAS FLOW MEASUREMENT**

VERBESSERUNGEN AN ODER IM ZUSAMMENHANG MIT EINER GASSTROMMESSUNG

PERFECTIONNEMENTS APPORTÉS OU SE RAPPORTANT À LA MESURE D'UN DÉBIT GAZEUX

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.06.2015   GB 201511360**

(43) Date of publication of application:
**02.05.2018   Bulletin 2018/18**

(73) Proprietor: **Oxford University Innovation Limited
Oxford, Oxfordshire OX2 0JB (GB)**

(72) Inventors:
• **ROBBINS, Peter Alistair
Oxford
Oxfordshire OX1 3PT (GB)**
• **O'NEILL, David
Oxford
Oxfordshire OX1 3QZ (GB)**

• **COUPER, John
Oxford
Oxfordshire OX1 3QZ (GB)**
• **HANCOCK, Gus
Oxford
Oxfordshire OX1 3QR (GB)**
• **RITCHIE, Grant
Oxforfd
Oxfordshire OX1 3QR (GB)**
• **CIAFFONI, Luca
Oxford OX1 3QR (IT)**

(74) Representative: **J A Kemp LLP
14 South Square
Gray's Inn
London WC1R 5JJ (GB)**

(56) References cited:
**EP-A1- 2 404 552      US-A- 3 504 542
US-A1- 2009 056 472**

**Description**

The present invention relates to an improved gas flow meter and to the use of the improved gas flow meter as a means of measuring molecular flow, and in particular enabling measurement of gas exchange by a respiring subject, including oxygen consumption and carbon dioxide production and the selective up-take of diagnostic gases.

Measurement of a gas flow, for example in litres per unit time, does not directly give the molecular flow (i.e. the number of molecules of a target gas in the flow) because the molecular flow depends on the pressure and temperature of the gas and, in a mixture, the fractional content of the target gas. The molecular flow can be calculated for a flowing gas mixture if one knows either the concentration of the target gas and the gas flow, or the fraction of the target gas together with temperature, pressure and flow.

The accurate measurement of oxygen consumption and carbon dioxide production in a respiring human or animal subject is useful for assessment of both cardiopulmonary and metabolic function. Both measurements are useful in medical or veterinary applications such as during anaesthesia, or artificial or assisted ventilation of a subject including within the setting of critical care, and during exercise stress testing which has both medical and sports applications. The accurate measurement of gases such as methane, acetylene, carbon monoxide and nitrous oxide is also useful in diagnosis and anaesthesia.

The need for accurate measurement of molecular flow also arises in industrial applications such as analysis of combustion products from engines or incinerators for example, measurement of consumption of gaseous reactants or gaseous reaction products in chemical reactors.

To measure molecular flow accurately therefore both the concentration of the target gas and the gas flow need to be measured accurately.

Currently there are a variety of ways of measuring oxygen concentration. For example, oxygen sensors based on electrochemical cells, polarography, paramagnetism and zirconium oxide are all known for detecting oxygen, but these are not ideal for respiratory applications as generally they have to be positioned remotely from a subject because they are unsuitable for inclusion within a ventilation tube or airway, and they also have a relatively slow response time. Measurements of carbon dioxide have generally used infrared absorption (capnography) which although being more amenable to in-line monitoring, has generally also been employed in a side-stream configuration. Mass spectrometers have also been used to measure both the oxygen and carbon dioxide concentration in breath, but because of their large size and weight, again they need to be positioned remotely from the patient. Furthermore, they generally require a sample at, or near, atmospheric pressure to be expanded into a lower pressure region for detection. Mass spectrometers are also relatively expensive and delicate and not particularly suitable for use in the average clinical, sports or industrial environment.

Nevertheless, analysers based on these technologies are routinely used in monitoring oxygen and carbon dioxide in breath, for example during anaesthesia, but the positioning of the analyser remotely from the patient means that it is not possible to produce concentration measurements that can be temporally aligned with sufficient accuracy with flow measurements to calculate oxygen consumption and carbon dioxide production by direct integration of the product of concentration and flow over time. In general the analyser is connected to the breathing tube of the patient by a catheter and a pump is used to transport gas through the catheter to the analyser and this introduces variable delay times in the arrival of gas at the analyser. There may also be further problems with the concentration measurement in that there can be longitudinal mixing of the gas in its journey from the breathing tube to the analyser, making the concentration measurement less accurate. Thus, analysers used in this configuration (sampling gas via a catheter from a patient's breathing tube) can provide the temporal profile of the carbon dioxide and oxygen in the breathing tube, but not in a way that can be used to determine oxygen consumption and carbon dioxide production with any degree of accuracy.

A way of working around this problem to some extent has been to connect the patient's breathing tube as a branch from a high (fixed) flow of gas supply of a known fixed composition (almost always air). The expired gas from the patient mixes back with the high flow gas supply and the two are fed to a chamber where they are homogenised by a fan and the concentration of oxygen is measured. The fan chamber is large enough to produce a constant concentration of oxygen and the average oxygen consumption can be calculated by multiplying the fixed high flow of gas by the difference between the original oxygen concentration in the incoming gas and the oxygen concentration value in the mixing chamber. However, the method relies on the use of the fan chamber to smooth out time-dependent variations in molecular concentrations and thus the temporal resolution of the system is inherently poor. It is therefore impossible to calculate oxygen consumption and carbon dioxide production on a breath-by-breath basis. The system also will not function if the patient is breathing anything other than a fixed gas composition, so is not suitable for use in anaesthesia where low (variable) gas flows may also be required.

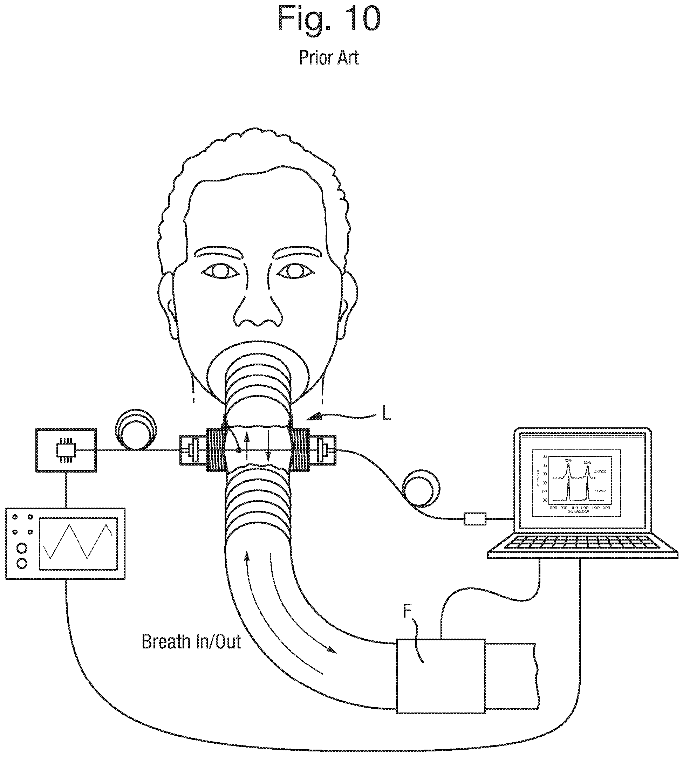

Figure 10 of the accompanying drawings illustrates an apparatus for measuring gas concentrations in breath as disclosed in WO-A1-2010/058150. It improves on the currently available breath analysis techniques by positioning a laser gas analyser L directly in the airway close to the patient's mouth, the laser gas analyser including an optical cavity for detecting oxygen by cavity-enhanced absorption spectroscopy (CEAS) and a light source and detector for carbon dioxide measurement by either direct absorption spectroscopy or wavelength/frequency modulation spectroscopy. The gas flow in the airway is measured using a conventional flow meter F positioned in a separate region down the airway from the laser gas analyser. Although this arrangement provides an improvement on what has been previously proposed and used, the arrangement still does not ensure that the flow sensing and gas analysis occur under the same physiochemical conditions, as the gas may change temperature and water vapour may condense between the two sensors. This would introduce inaccuracies into the molecular flow sensing.

Under conditions of air breathing, the rate of oxygen consumption ($\dot{V}o_2$) can be determined relatively easily in humans by measuring the volume of gas breathed out over a number of breaths, and determining its oxygen and carbon dioxide, and hence nitrogen, concentration. From this, the amount of oxygen and nitrogen that has been breathed out is obtained. As the composition of air is known, the amount of oxygen breathed in can be calculated by finding the volume of air that contains the equivalent volume of nitrogen as has been breathed out. The difference between the volume of oxygen breathed in and that breathed out gives $\dot{V}o_2$. This method of calculating oxygen consumption from expiratory measurements using nitrogen balance is known as the Haldane transformation, and it forms the basis of most methods for determining oxygen consumption in areas such as cardiopulmonary exercise testing.

When the inspired oxygen fraction is elevated, as it is often the case in anaesthesia and intensive care, current approaches to measuring oxygen consumption work much less well. Knowledge of the inspired fractions for oxygen and nitrogen becomes less accurate when the inspired gas cannot be assumed to be air; the breath to breath variation in lung stores of oxygen becomes more significant as the oxygen fraction increases; and the oxygen consumption itself becomes a smaller and smaller fraction of the total tidal flow of oxygen, thus increasing error. In a study of existing technologies in critical care, Black et al. (C. Black, M. P. W. Grocott, M. Singer, Metabolic monitoring in the intensive care unit: $\alpha$ comparison of the Medgraphics Ultima, Deltatrac II, and Douglas bag collection methods, Br. J. Anaesth. 114, 261-268 (2014)) concluded: "For this field to move forwards, industry must collaborate with clinicians and researchers to improve the accuracy of devices that monitor gas exchange in mechanically ventilated patients."

In practice, measuring oxygen consumption when the inspired oxygen is high involves subtracting the large volume of oxygen breathed out from the large volume of oxygen breathed in, in order to determine a relatively small number. Thus, small errors in either measurement relative to the other will result in large errors in oxygen consumption. By way of example, consider a subject breathing pure oxygen with an inspired pulmonary ventilation of 5 l/min. If the oxygen consumption $\dot{V}o_2$ (STPD; Standard Temperature and Pressure, Dry) is 0.25 l/min, then the expired oxygen flow will be 4.75 l/min. If a 5% error in the measurement of $\dot{V}o_2$ is acceptable (equivalent to 0.0125 l/min), then the measurement of oxygen breathed in and out must be accurate to 0.25%. For comparison, current standards for accuracy for measurement of respiratory volumes are $\pm$ 3%.

Mathematically, determining $\dot{V}o_2$ (STPD) is equivalent to evaluating the integral:

$$\dot{V}_{O2} = \frac{k}{\Delta t} \int \dot{V} F_{O2} dt$$

where $\Delta t$ is the period of integration and $k$ is a constant (dependent upon pressure and temperature) to convert to STPD; $\dot{V}$ and $F_{O2}$ are the respiratory flow and fractional concentration of oxygen at time t, respectively. In order to achieve the target level of precision, it is necessary to ensure that the instantaneous concentrations and flows in Equation 1 are perfectly time-aligned, and that both measurements are made using techniques which have sufficient precision and sufficiently rapid dynamic responses to follow the changes accurately during a breathing cycle.

US 2009/056472 A1, , on which the precharacterizing portion of claim 1 is based, discloses an adaptor for a flow sensor. EP2404552 A1 discloses a system and method for aiding in the diagnosis of a respiratory dysfunction.

Accordingly the present invention provides a molecular flow sensor as defined by the appended claims.

By integrating the gas flowmeter and gas composition analyser, the gas flow and gas composition is measured in the same measurement space. This means that there are no time differences between the flows being measured and the gas compositions being measured, that both measurements are made at the same temperature and pressure, and also that measurements of gas viscosity and density obtained from the gas composition analyser can be used to correct the flow measurements.

Preferably the gas composition analyser is configured to measure the gas concentrations very frequently in order to follow rapid changes in the gas composition. Measurements are preferably made at least every hundred milliseconds, preferably more frequently than every 50 milliseconds, for example about every 10 milliseconds. Preferably the flow measurements are made at the same rate.

Preferably the flowmeter is of the Lilly type in which the speed of flow of gas is measured by monitoring the pressure drop across a mesh, referred to herein as a flow-sensing mesh. US-B-5,060,655 discloses a pneumotachometer in which a flow-sensing mesh with approximately 120-130 openings per cm is placed between two flow-conditioning meshes having a lower flow resistance (between 40 and 50 openings per cm) with the pressures on either side of the assembly of meshes being sensed/measured. With the present invention, however, preferably the flowmeter comprises two flow-sensing mesh screens spaced along the flow path of gas. The term "flow-sensing mesh screen" is intended to mean a mesh component designed to create a detectable pressure drop in gas flowing through it. The detection of the pressure drop allows calculation of the gas flow speed. The mesh size used depends on the gas and the flow speed which is to be detected. In the applications intended for the present invention mesh with wire diameter from 25μm to 90 μm and gaps between wires from 50 μm to 180 μm, depending on the anticipated flow, are suitable. Flow-sensing mesh screens differ from flow conditioning components, as flow conditioning components are generally of lower flow resistance and are intended to condition the gas flow, e.g. by removing turbulence, making the flow laminar, smoothing the flow, rather than creating a measurable pressure drop.

The first of the two flow-sensing mesh screens is located upstream of the measurement space and the second is located downstream of the measurement space. A differential pressure sensor is provided for measuring the pressure difference between the upstream side of the first flow-sensing mesh screen and the downstream side of the second flow-sensing mesh screen. In the present specification the terms "upstream" and "downstream" are with respect to the direction of gas flow through the airway. In a respiratory application as the gas flow reverses with inhalation and exhalation what is regarded as upstream or downstream for exhalation will become downstream or upstream respectively for inhalation.

With the present invention the positioning of the two flow-sensing mesh screens either side of the measurement space contributes to the accurate time alignment of the measurements of gas composition and gas flow. Furthermore, the accurate time alignment means that measurements of the gas composition can be used to correct the gas flow measurements for variations in composition, viscosity and density.

Preferably the two flow-sensing mesh screens are identical to each other so that the flowmeter is accurate for measurement of bidirectional flow.

Preferably the differential pressure sensor is connected to the flow path via respective pressure sensing ports, one on an upstream side of the first of the two flow-sensing mesh screens and the other on a downstream side of the second of the two flow-sensing mesh screens.

Preferably the molecular flow sensor includes at least two identical flow conditioning elements respectively and symmetrically positioned upstream and downstream of the upstream and downstream pressure sensing ports. The flow conditioning elements may comprise flow conditioning meshes, for example of lower flow resistance than the flow-sensing meshes, for example of 100 μm stainless steel wire with a 250 μm gap between wires. The flow conditioning elements may instead or in addition comprise, helical or otherwise, baffles forming a non-straight flow path for the gas. Preferably the baffles also form a light trap which prevents light from the airway from entering the measurement space. The flow conditioning elements may alternatively or additionally comprise a metal foam, such as nickel foam.

Each of the pressure sensing ports is preferably connected to a pressure averaging chamber which communicates with the gas flow path at a plurality of locations circumferentially spaced around the flow direction. Thus the potentially different gas pressures at the plurality of locations around the flow path are physically averaged together in the pressure averaging chamber, and the chamber is in gas communication with the differential pressure sensor. The plurality of paths connecting the pressure averaging chamber to the respective locations preferably have a flow resistance which is high compared with that of the pressure averaging chamber. This ensures that pressures distal to the pressure sensor are weighted equally with pressures close to the pressure sensor. The communication between the pressure averaging chamber and the gas flow path may be achieved by providing a pressure sampling hole at each of said plurality of locations around the flow path, each pressure sampling hole communicating with the pressure averaging chamber. Alternatively an annular slit may be provided which extends through the plurality of locations around the flow path to provide communication between the pressure averaging chamber and the gas flow path.

Preferably the pressure averaging chamber is an annular channel circumferentially surrounding the gas flow path.

A single differential pressure sensor is provided connected to the pressure sensing port upstream of the measurement space and also to the pressure sensing port downstream of the measuring space.

Preferably all components influencing the gas flow path are symmetrically disposed either side of the measurement space and are identical so that accurate measurements are provided for bidirectional flow.

Preferably the molecular flow sensor further comprises a temperature sensor for sensing the temperature of gas in the flow path. In one embodiment the temperature sensor comprises a plurality of individual temperature detectors such as thermocouples, at least one positioned axially of the flow path and at least one positioned off axis of the flow path. The use of plural temperature detectors both on-axis and off-axis provide effective temperature averaging. In a second embodiment, the averaging can be carried out by using a spectroscopic method of temperature sensing, where the relative intensities of two or more spectral features are measured, with the relative intensities being temperature dependent for example because of temperature dependent populations in the individual quantum levels probed.

Preferably the gas composition analyser is an absorption spectrometer for measuring the absorption of light along optical paths through the measurement space. One form of absorption spectrometer is a cavity enhanced absorption spectrometer (CEAS) which utilises an optical cavity enclosing the measurement space. The optical cavity is formed by highly reflective mirrors and has its optical axis perpendicular to the flow path. The use of such an optical cavity increases the sensitivity of the spectrometer allowing detection of weakly absorbing species, such as from molecular oxygen. The gas composition analyser may also include absorption or modulation spectrometers for the detection of respiratory and other gases such as carbon dioxide and water, CO, NO, acetylene, methane, nitrous oxide, and these may be positioned to use separate optical paths or the same optical path; both cases preferably utilise optical paths which are distinct from that/those traversed by light propagating within the optical cavity.

The molecular flow sensor comprises a processor to receive the differential pressure measurements, the gas composition or spectral measurements and preferably also the absolute pressure and temperature of gas and adapted to calculate the flow of all gas species analysed together with the flow of the balance gas. Preferably, the flow measurements include those for oxygen carbon dioxide and water to enable the calculation of oxygen consumption and carbon dioxide production.

In a setting where individual gas flows are not required and variations in density and viscosity may be neglected, an alternative aspect to the invention provides for a pneumotachometer for measuring flow of respiratory gas along an airway, the pneumotachometer comprising: two identical flow-sensing mesh screens spaced along the flow path of respiratory gas through the airway; a differential pressure sensor connected to the airway to sense, via respective pressure-sensing ports, the difference in pressure between an upstream side of the first of the two flow-sensing mesh screens and a downstream side of the second of the two flow-sensing mesh screens; the pneumotachometer further comprising: at least two identical flow-conditioning elements respectively and symmetrically positioned upstream and downstream of the upstream and downstream pressure-sensing ports.

The flowmeter comprises two flow-sensing mesh screens spaced along the flow path of gas. In the applications intended for the present invention mesh with wire diameters from $25\mu m$ to $90 \mu m$ and gaps between wires from $50 \mu m$ to $180 \mu m$, depending on the anticipated flow, are suitable. Preferably the two flow-sensing mesh screens are identical to each other so that the flowmeter is accurate for measurement of bidirectional flow.

A differential pressure sensor is provided for measuring the pressure difference between the upstream side of the first flow-sensing mesh screen and the downstream side of the second flow-sensing mesh screen. The differential pressure sensor is connected to the flow path via respective pressure sensing ports, one on an upstream side of the first of the two flow-sensing mesh screens and the other on a downstream side of the second of the two flow-sensing mesh screens.

Preferably the molecular flow sensor includes at least two identical flow conditioning elements respectively and symmetrically positioned upstream and downstream of the upstream and downstream pressure sensing ports. The flow conditioning elements may comprise flow conditioning meshes, for example of lower flow resistance than the flow-sensing meshes, for example a mesh of $100 \mu m$ diameter wire with a gap of $250 \mu m$ between wires. The flow conditioning elements may instead or in addition comprise, helical or otherwise, baffles forming a non-straight flow path for the gas.

Each of the pressure sensing ports preferably comprises a pressure averaging chamber which communicates with the gas flow path at a plurality of locations circumferentially spaced around the flow direction. The plurality of paths connecting the pressure averaging chamber to the respective locations preferably have a flow resistance which is high compared with that of the pressure averaging chamber. The communication between the pressure averaging chamber and the gas flow path may be achieved by providing a pressure sampling hole at each of said plurality of locations around the flow path, each pressure sampling hole communicating with the pressure averaging chamber. Alternatively an annular slit may be provided which extends through the plurality of locations around the flow path to provide communication between the pressure averaging chamber and the gas flow path.

Preferably the pressure averaging chamber is an annular channel circumferentially surrounding the gas flow path.

A single differential pressure sensor is provided connected to the pressure sensing port upstream of the measurement space and also to the pressure sensing port downstream of the measuring space.

The invention will be further described by way of examples with reference to the accompanying drawings in which:-

Figure 1 schematically illustrates a respiratory gas analyser in accordance with an embodiment of the present invention;
Figure 2A schematically illustrates the optical paths for one embodiment of the respiratory gas analyser and Figure 2B schematically illustrates the gas analyser measurement head of one embodiment of the invention;
Figure 3 schematically illustrates the gas flow path components according to one embodiment of the invention;
Figures 4A-D schematically illustrate one example of the pressure sensing ports;
Figure 5 schematically illustrates two pressure sampling ports connected to a single differential pressure sensor in accordance with one embodiment of the invention;

Figure 6 schematically illustrates an example without being part of the invention in which plural pressure sampling ports are connected to respective distributor manifolds and plural differential pressure sensors in an alternative embodiment of the invention;

Figures 7(A) to (D) show transmission spectra for oxygen, carbon dioxide and water measured by an embodiment of the invention fitted by a model and, in the subplots, residuals from the fitted model and Figures 7 (D) to (F) show calibration curves;

Figure 8 illustrates oxygen, carbon dioxide and water concentration outputs measured by an embodiment of the invention in a typical oxygen wash-in experiment;

Figure 9 illustrates the respired gas flows calculated for the oxygen wash-in experiment of

Figure 8;

Figure 10 schematically illustrates a prior art breath analysis apparatus;

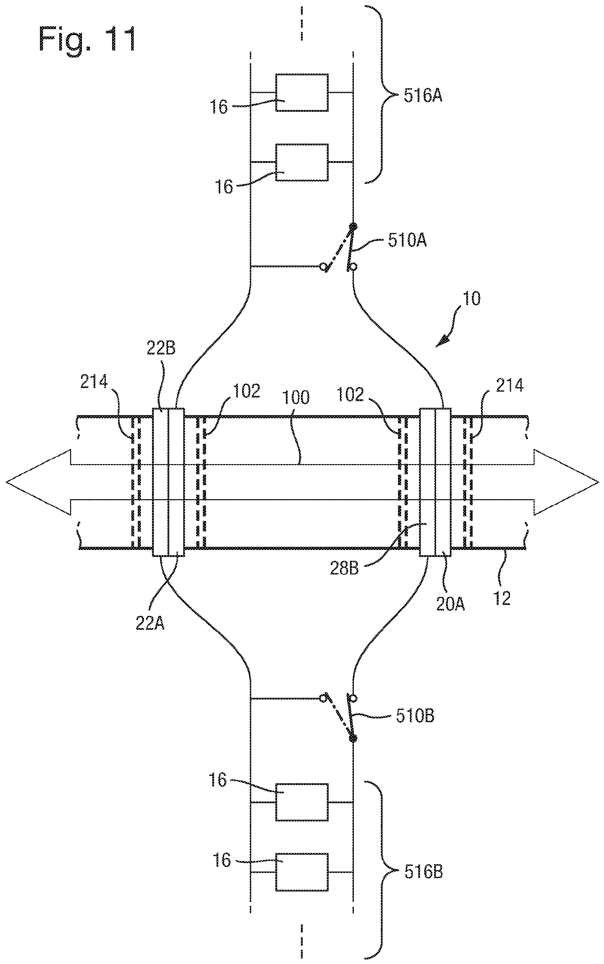

Figure 11 schematically shows an example not being part of the invention; and

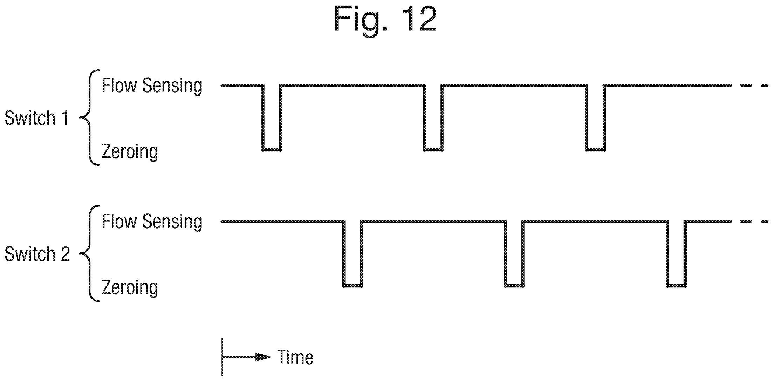

Figure 12 illustrates one example of the timing of valve switching in the example of Figure 11.

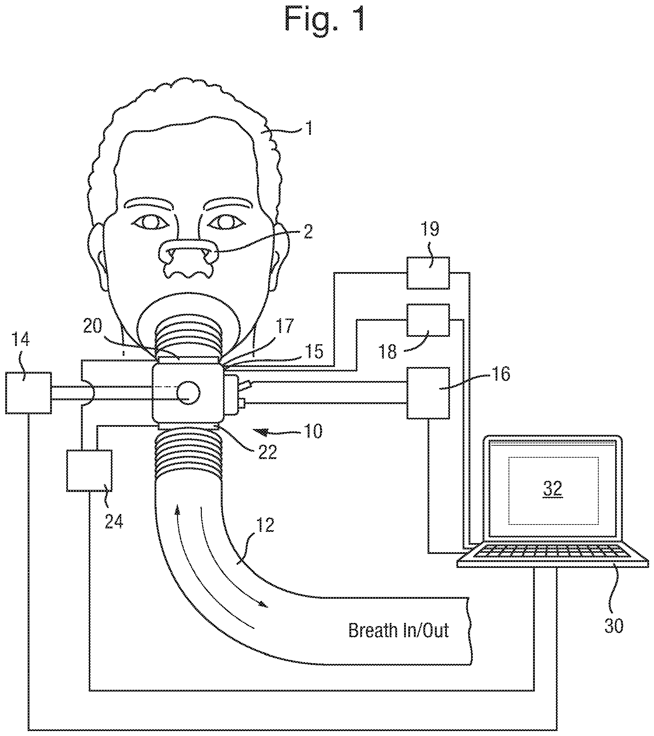

Figure 1 schematically illustrates a molecular flow sensor in accordance with one embodiment of the invention used inline in an airway to measure respiratory gases. The main functional components are in a measurement head 10 positioned in an airway 12 of a patient 1. In a critical care environment the airway may be connected to the patient's air and/or oxygen supply or during anaesthesia to a supply of air and/or oxygen and anaesthetic. In exercise monitoring the airway may be simply open to atmosphere.

The measurement head 10 is connected to a controller 14 for controlling the cavity enhanced absorption spectrometer within the measurement head used, in this embodiment, for oxygen concentration measurement, and a controller 16 is provided for controlling the light source and light detector for a separate optical path used for carbon dioxide and water concentration measurements. On either side of the measurement head 10 are pressure sampling assemblies 20 and 22 connected to a differential pressure sensor 24. The measurement head 10 also includes a temperature sensor 15 for measuring the temperature of the gas, and a barometric pressure sensor 17 for measuring the static pressure inside the measurement space, connected to respective controllers 18 and 19. The temperature sensor 15 may be formed by using multiple thermocouples, positioned both axially and off axis, so as to provide average effective temperatures for the gas analysis and for the flow as described below. An alternative embodiment measures temperature over the measurement space (the flow channel) using spectroscopic means, for example, in the determination of temperature dependent quantum state populations, with high time resolution using for example, a multi-mode Fabry Perot diode laser.

The individual controllers 14, 16, 18, 19 and 24 are connected to a system controller 30 which controls the individual controllers, receives their outputs and calculates and displays the results on a display 32.

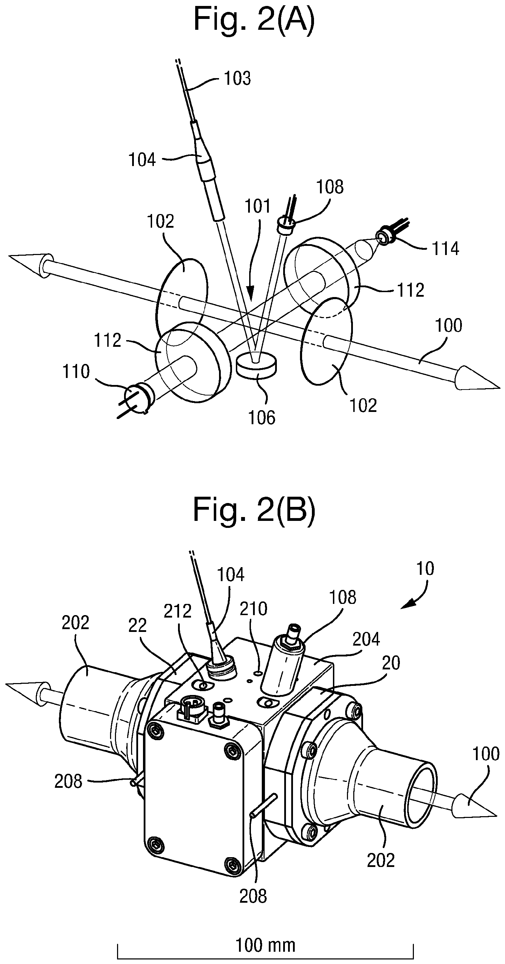

Figure 2 schematically illustrates the measurement head 10 in more detail. Figure 2A illustrates the optical components and optical paths in relation to the gas flow path and Figure 2B illustrates an external view of the measurement head 10. As illustrated in Figure 2A, to measure the speed of flow of gas two stainless steel flow-sensing mesh screens 102 are located along the straight, bi-directional gas flow path 100. In this particular embodiment the flow-sensing mesh screens 102 preferably are of woven stainless steel have a wire diameter of 35 $\mu$m and a gap between wires of 75 $\mu$m in order to give a detectable pressure drop of around 2 millibar across both meshes in series for flows of around 4 litres per second. In the applications intended for the present invention, mesh with wire diameter from 25$\mu$m to 90 $\mu$m and gaps between wires from 50 $\mu$m to 180 $\mu$m have been used, depending on the anticipated flow rate. The pressure drop across the two flow-sensing mesh screens 102 is measured by use of one or more differential pressure sensors connected to the gas flow path 100 on opposite sides of the flow-sensing mesh screens from the measurement space, i.e. upstream of the upstream mesh screen and downstream of the downstream mesh screen as explained in more detail below.

As illustrated in Figure 2A, integrated within the flow meter is a laser absorption spectrometer for measuring the gas composition within the measurement space 101 between the two flow-sensing mesh screens 102. The laser absorption spectrometer in this embodiment comprises three detection channels each composed of a laser diode light source, photo detector and optical path along which the molecular gas concentration is measured. The optical wavelength of each laser diode light source is repeatedly scanned across a narrow region of the electromagnetic spectrum that exhibits an absorption signature specific to the species being measured, in this case oxygen, carbon dioxide or water. Although three species are to be measured, in this embodiment the spectrometer has only two separate optical paths for measuring all three species because the detection channels dedicated to carbon dioxide and water have similar path length requirements and so share a common optical path. In this embodiment, two fiber-coupled diode lasers are used to probe the absorption features exhibited by carbon dioxide and water vapour at 2004 nm and 1368 nm respectively; both species may be probed using two of a number of absorption transitions in the infrared portion of the electromagnetic spectrum. Their outputs are spatially combined by a fiber optic multiplexer and guided through an umbilical hybrid cable 103 into the measurement head 10. A fiber optic collimator 104 launches the radiation into a V-shaped light path reflected off a concave mirror 106 to a single detecting photo diode 108 which sends its output via controller 16 to system controller 30. The transmitted spectra associated with carbon dioxide and water are, in this embodiment, temporally de-multiplexed by scanning the two lasers alternately within the measurement time window.

In this embodiment, the spectrometric channel for measuring oxygen concentration is located transversely of the gas flow path 100. Light emitted by a 764 nm, low power laser diode 110 is launched into a 27 mm optical cavity constructed from a pair of highly reflective cavity mirrors 112. As explained in WO-A-2010/058150 the cavity enhancement achieves a significant increase in the effective interaction path length between the light and the oxygen in the respiratory gas and consequently in the magnitude of the oxygen absorption signal. The cavity mirrors 112 have a reflectivity greater than 99% and thus some light emerges from the optical cavity to be detected by a photodiode 114 on the side opposite to the laser diode 110. The output signal from the photodiode 114 is preferably amplified, for example by a high-gained trans-impedance amplifier and an output to a controller 14 and in turn to the system controller 30.

The high sensitivity of the detector 114 means that ambient light must be prevented from entering the measurement space 101. In this embodiment this is achieved by providing helical light traps upstream and downstream of the measurement space as described in more detail below.

The measurement space 101 has a diameter of about 24 mm and is, overall, particularly compact, having a volume of about 20 cm$^3$.

Figure 2B schematically illustrates the measurement head 10 in its assembled configuration. Standard cone and socket airway connectors 202 which directly interface with standard 22 mm respiratory equipment connectors are provided on the upstream and downstream sides of the measurement head. The differential pressure sensor(s) are connected across the differential pressure sensing ports 208 which are upstream and downstream of the flow measurement meshes 102 defining the measurement space 101. The connectors 104 and 108 for the V-path spectrometric channel are provided on the top (as illustrated) of the housing 204 and the housing 208 for the cavity mirror and light source for one side of the optical cavity is provided on one side of the housing 204. The housing 204 is also provided with a pressure port 210 for the barometric pressure sensor 17 which provides a measurement of the barometric pressure within the airway, and also with a connector 212 for connection to a plurality of temperature detectors (in this case four) provided around the measurement space 101 which provides the temperature sensor 15 giving a measurement of the temperature of the respiratory gas. In this embodiment, four 25 micron Type K thermocouples are positioned within the measurement space. In this embodiment the temperature detectors are positioned close to the internal surface of the flow-sensing mesh screens. One of each pair is positioned axially and the second close to the circumference of the measurement space.

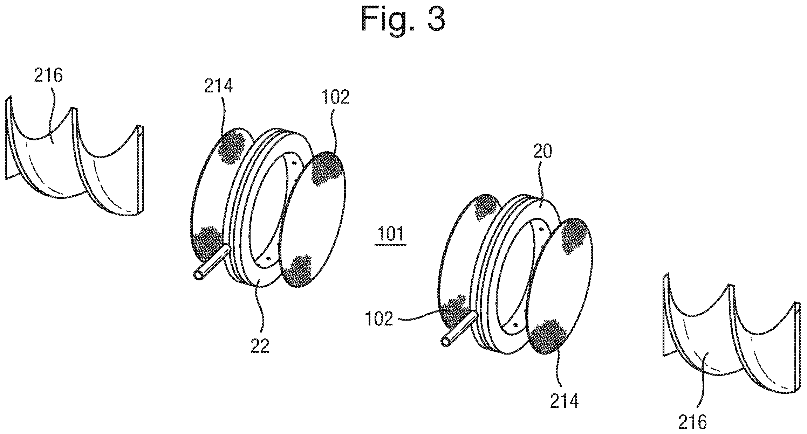

Figure 3 illustrates the flow path components in more detail. The measurement space 101 is defined between the two flow-sensing mesh screen 102. The static pressures upstream and downstream of the flow-sensing mesh screens 102 are sampled by use of respective pressure sampling assemblies 20 and 22. Because the pressure drop across the flow-sensing mesh screens 102 depends to an extent on upstream flow geometry, the measurement head includes flow conditioning components. Firstly, a pair of additional flow-conditioning mesh screens 214 are provided upstream and downstream respectively of the upstream and downstream pressure sampling assemblies 20, 22. These mesh screens are of 100 μm stainless steel wire with a 250 μm gap between wires. In an alternative embodiment porous metal foam elements may be used rather than meshes. For example, a nickel foam a few mm thick with a sub-millimeter pore size, e.g a 3mm thick foam with a mean pore size of 0.6mm such as Ni-2733 from Recemat BV. Similar porous foams with larger or smaller pores and which are thicker or thinner can be used as long as they condition the flow of gas.

To further condition the gas flow and to act as light traps a pair of light baffles 216 are also provided upstream and downstream of the flow-conditioning mesh screens 214. These baffles form a curved flow path for the gas, but with a very low flow resistance. They also block any ambient light from entering the measurement space 101. They are made from a material selected to have a low reflectivity over the optical wavelength range to which the detectors are sensitive and have a geometry such that they block all direct light paths between the measurement space and the outside of the instrument. Preferably the baffles define a helical flow path for the gas.

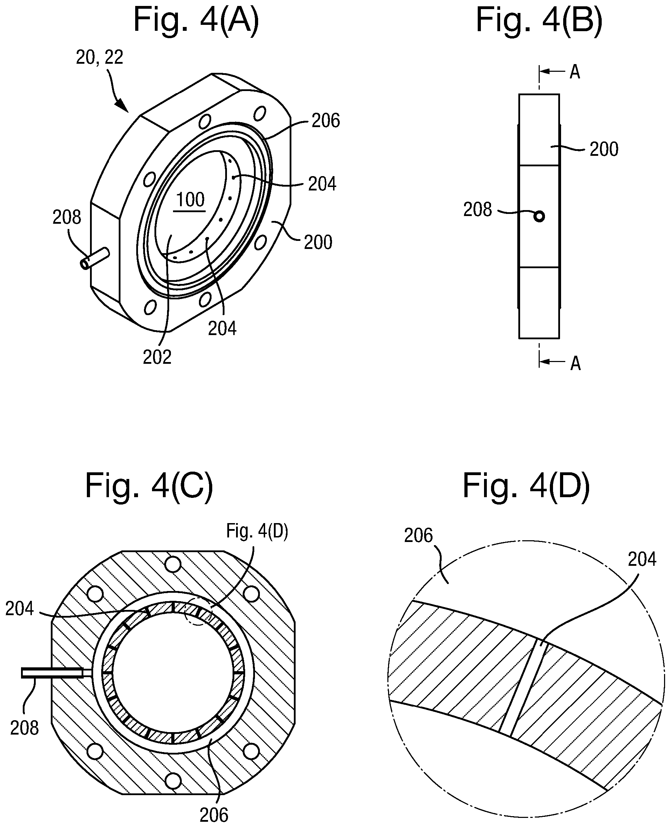

Figure 4 illustrates one of the pressure sampling assemblies 20, 22. The assembly is illustrated isometrically in Figure 4A and consists of an outer annulus 200 through which a circular orifice 202 is formed to provide the gas flow path 100. An array of pressure sampling holes 204 extend from the orifice 202 to an annular pressure averaging channel 206 which circumferentially surrounds the flow path 100. The pressure sampling holes (for example there are 16 holes in the illustrated example) are typically of diameter from 100 to 400 μm, more typically 250 μm, and they form a relatively high resistance compared to the relatively lower resistance pressure averaging channel 206. The pressure averaging channel 206 is provided with a port 208 for connection to one side of a differential pressure sensor. Figure 2B illustrates the pressure sampling assembly 20, 22 in side elevation and Figure 4C illustrates a cross-section of the pressure sampling assembly along line A-A of Figure 4B. Figure 4D illustrates the detail of one of the pressure sampling holes 204 from section A-A of Figure 4C.

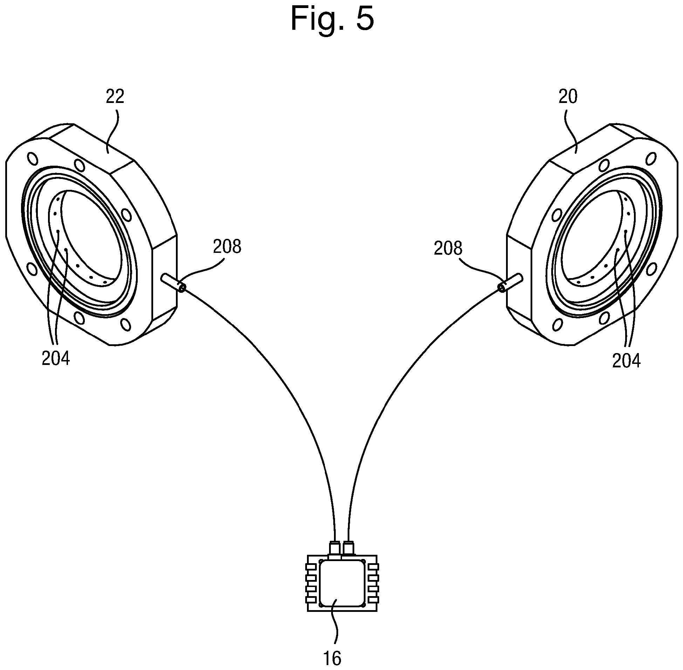

Figure 5 illustrates the connection of the two pressure sampling ports 208 to a differential pressure sensor 16 such as a ±2.5 millibar piezoelectric membrane differential pressure sensor (e.g. by Sensortechnics). In operation one of the pressure sensing assemblies 20 is upstream of the measurement head 10 including the two flow-sensing mesh screens and the other, for example 22 is downstream of it (with respect to the gas flow at the time). The differential pressure sensor 16 outputs a reading which is dependent upon the difference in static pressure at the two pressure sensor ports 208. This pressure is physical average of the pressures contributed to the annular pressure averaging channel by the array of pressure sensing holes 204.

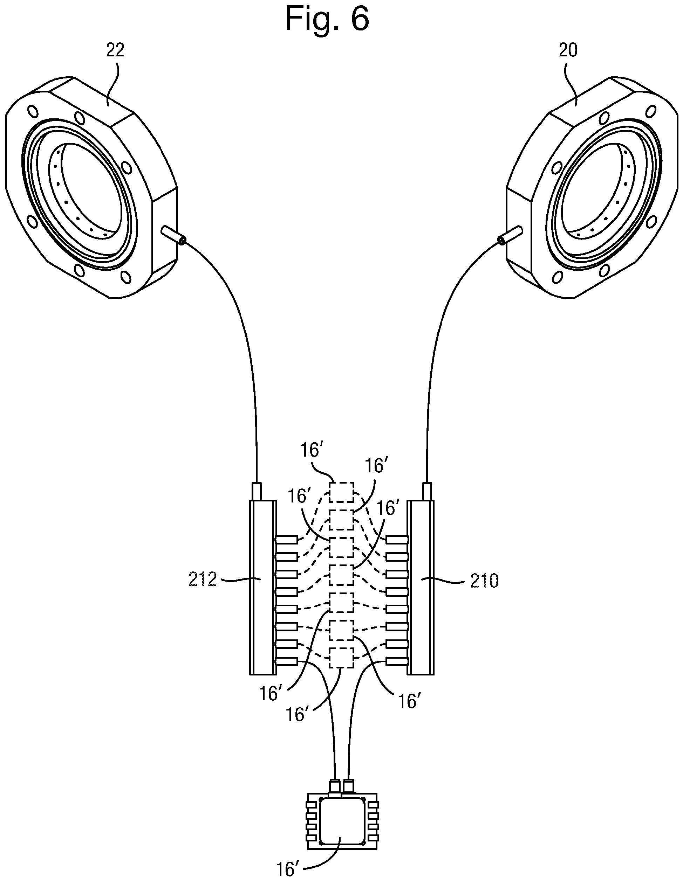

Although the sensor-to-sensor variation of zero flow reading for this type of differential pressure sensor 16 is small, it may nevertheless be significant with respect to the tolerances required in the present application. If necessary to improve the accuracy, therefore, in an an example not being part of the invention an array of eight differential pressure sensors 16' may be used, each connected via an 8-way manifold 210, 212 to the two pressure-averaging chambers as illustrated in Figure 6. Four of these sensors are connected in one polarity and four using the opposite polarity to reduce the effects of any intrinsic asymmetry in the sensors. The pressure readings obtained from the eight differential pressure sensors 16' are then averaged by the controller 24 or by the system control unit 30.

To reduce problems created by the high water vapour content of exhaled breath, a standard, disposable, medical-grade heat and moisture exchanging (HME) filter may be located between the patient 1 and the input side of the measurement head 10. This both lowers the temperature of the gas entering the measurement space during exhalation and reduces its water vapour content. In addition to eliminate problems caused by condensation, the measurement space is heated to a constant temperature of about 34°C by a heater (not illustrated).

Figure 11 schematically illustrates another example without being part of the invention in which two independent differential pressure sampling systems are provided so that either one of them can be switched out at any time for zero point calibration while the other continues to monitor the differential pressure. Thus with the second example not being part of the invention, continual measurement of flow is possible, even though one of the pressure sampling systems is being calibrated. This is useful in the case of differential pressure sensors 16 which have unpredictable drift and thus need to have a relatively recent "zero" value read in order to give accurate differential pressure values. By having two independent pressure sampling and measurement systems, each can be isolated from the differential pressure signal at a different time point allowing the system controller to take the read-outs from the differential pressure sensors with zero pressure drop and update the zero offset for that sensor.

In more detail, as illustrated in Figure 11, each of the pressure sampling assemblies comprises a first and second assembly 20A, 20B and 22A, 22B, each of which can be the same as the pressure sampling assemblies 20, 22 in the first embodiment. Thus each can include a pressure sensor manifold which connects via a pressure averaging channel 206 to two or more pressure sampling holes 204 communicating with the gas flow path 100 on one side, and via a pressure sampling port 208 to one or more differential pressure sensors 16 on the other side. Thus, as illustrated in Figure 11, pressure sensing assembly 20A is connected to a first bank 516A of differential pressure sensors 16 while pressure sampling assembly 20B is connected to a second bank 516B of differential pressure sensors 16. The pressure sampling assembly 22A is connected to the other side of the differential pressure sensors 16 forming the first sensor bank 516A, and the pressure sampling assembly 22B is connected to the other side of the differential pressure sensors 16 forming the second sensor bank 516B.

The first and second sensor banks 516A and 516B are connected to the respective pressure sampling assemblies 20A and 20B by respective switch valves 510A and 510B. Each switch valve 510A and 510B allows its sensor bank to be switched between the normal pressure sampling and measuring mode (schematically illustrated with the solid position of the switch valve), and a pneumatic "short circuit" mode (illustrated dotted) in which both sides of the sensor bank are connected together and to only one of the pressure sensing assemblies (it does not matter which). As illustrated, with the switch valve 510A in the pneumatic "short circuit" position, both sides of sensor bank 516A are connected to pressure sensing assembly 22A. Similarly, with switch valve 510B in its pneumatic "short circuit" position, both sides of sensor bank 516B are connected to pressure assembly 22B. Thus both sides of the pressure sensors should read the same pressure when in the pneumatic "short circuit" mode, allowing any zero offset to be read and calibrated for. Although the switch valves 510A and 510B are illustrated as connecting to the pressure sensing assemblies 22A and 22B for the pneumatic "short circuit", they could connect to the pressure sensing assemblies 20A and 20B. All that is required is that both sides are connected to the same pressure. It should be noted that with the switch valves 510A and 510B in the short circuit mode, the disconnected pressure sampling assembly 20A or 20B is terminated in a pneumatic dead-end, i.e sealed.

As with the first example not being part of the invention, it is advantageous if the differential pressure sensors within each sensor bank include half connected with one polarity and half with the opposite polarity. Although two sensors 16 are illustrated in Figure 11 in each sensor bank, there may be more than two.

The switching of the sensor banks between pressure sensing and calibration can happen automatically at predetermined intervals, or under control of the system controller 30. In this example not being part of the invention, as illustrated in Figure 12, each switch valve is set, for most of the time, in the state where each side of the pressure sensor manifold 20A, B, 22A, B is connected to the appropriate sensor bank so that both of the pressure sensing systems are in the "flow sensing" state. The switching-out calibration period is a relatively short period (approximately 10 seconds out of 2-6 minutes) and as illustrated in Figure 12, the two switch valves 510A and 510B operate out of phase so that at any time at least one of the pressure sampling assemblies is connected to its differential pressure sensor bank. The switching-out does not need to be perfectly out of phase, as long as both sets of pressure sensors are not switched out at the same time.

In periods where a switching valve is isolating its sensor bank from the pressure sensing assembly, the controller 32 switches from using the signal from both sensor banks to only using the signal from the sensor bank which is not isolated from the pressure sensing assembly. At the same time the data from the isolated pressure sensor bank is used to determine new zero values for its differential pressure sensors.

In order to avoid any transient pressures associated with the switching operation of the switching valves interfering with the pressure measurements, the controller 32 ignores pressure readings for a certain predetermine time after a sensor bank is switched back in. For example, this may be one second, though the time can be adjusted depending on the type of switching valve used.

In operation the spectroscopic determination of the oxygen, carbon dioxide and water concentrations within the flowing breath sample involves repeatedly current-tuning the optical frequency of each laser diode across the absorption profile of the selected spectroscopic transition. Quantitative analysis of the frequency-dependent intensity signal recorded by each spectrometric channel is achieved by fitting to the experimental data a predicted shape of the transmission spectrum using regression. The sample temperature, barometric pressure and gas composition are needed for accurately reconstructing the spectral line shape of each absorption feature and the apparatus of this invention provides this information. In this embodiment, the rate at which the spectrometer updates the concentration outputs of the three measured gases (100 Hertz) is determined by the current tuning repetition rate set for each laser (200 Hertz) and by the number of consecutive transmission spectra averaged together before processing (two averages).

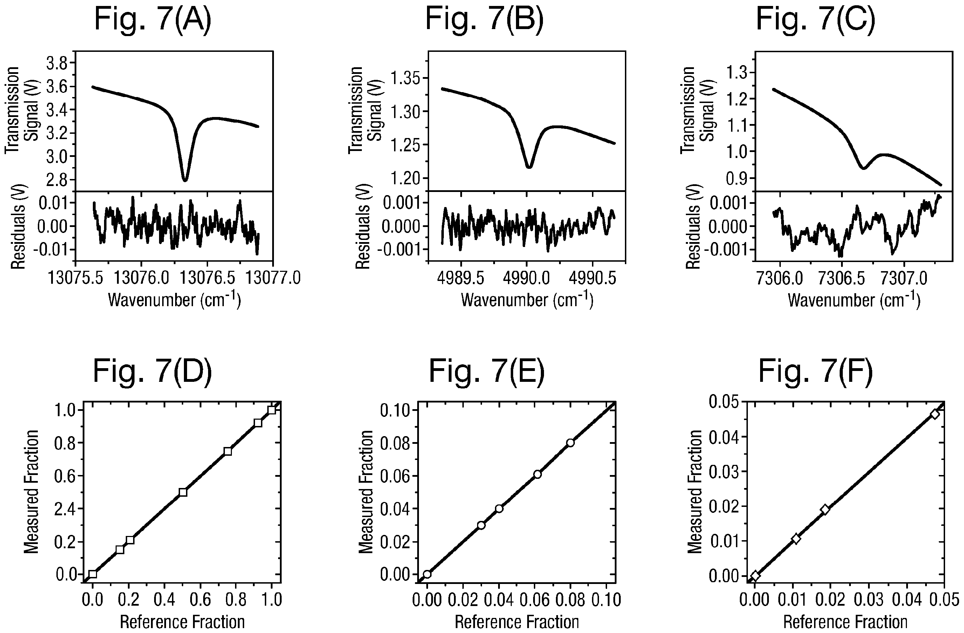

Figures 7A-C show examples of 10 millisecond transmission spectra for oxygen, carbon dioxide and water respectively, each superimposed with the best fitting theoretical transmission profile. The absence of systematic features in each of the residual's spectra shown in the subplots corroborates the high quality of the fitting procedure.

The concentrations of oxygen, carbon dioxide and water are calculated from the measured spectra in known fashion as described for example in WO-A-2010/058150. For example, for the CEAS scanning the laser frequency over the transition enables the calculation of:

$$S = \frac{I_0 - I}{I} = \frac{\sigma C L}{1 - R} \qquad (1)$$

where $I$ and $I_0$ are the intensities of the CEAS signals with and without absorption, respectively, $\sigma$ the wavelength dependent absorption cross section, $C$ the concentration of the species, $L$ is the separation of the mirrors and $R$ the geometric mean of the reflection coefficients of the two mirrors. From measurements of S the concentration C can thus be found. When the apparatus is switched on an initial calibration routine for the gas analyser is performed by using ambient air present in the cell (the fractional content of $O_2$ in a dry atmospheric sample is 20.95%) together with measurement of barometric pressure, temperature and the spectroscopic determination of water vapour content. This known concentration of oxygen is then used to calculate the value of $1/(1-R)$, which is then recalled by the data analysis routine in the system controller 30 for calculating the instantaneous $O_2$ concentration.

The time resolved direct absorption signals for carbon dioxide and water are converted into absolute concentrations via the Beer Lambert expression:

$$I(v) = I_0(v) e^{-\sigma(v) C L} \qquad (2)$$

This relationship states that the ratio of the intensities of radiation transmitted through the cell in the presence, $I(v)$, and absence, $I_0(v)$, of the sample, exhibits an exponential dependence on the concentration of the absorbing molecule, C, the path length travelled through the sample, $L$, and the absorption cross-section, $\sigma(v)$, describing the frequency-dependent probability of energy exchange between the probe radiation and the absorbing medium. If the physical path length and the spectral profile of $\sigma(v)$ are known (the latter being dependent upon the sample temperature, pressure, and composition), the controller 32 simply makes use of Eq..2 for calculating the molecular concentration without the need of prior calibration.

Investigation of the sensing performance for the $O_2$ and $CO_2$ spectra was conducted by sequentially flowing through the measurement cell, at a constant rate (~50 l/min), various known gas mixtures that had been gravimetrically-composed (BOC, UK, certified accurate within ±1% of the specified concentration value), and which covered a range of fractional contents of oxygen (0 - 100%), carbon dioxide (0 - 50%), and nitrogen (0 - 100%). With regards to the $H_2O$ channel, synthetic air was passed through a respiratory gas humidifier with the water bath set at various temperatures, and the partial pressure of water vapor in each sample flowing through the measurement cell was measured by a high-precision hygrometer based chilled mirror technology (Michell Instruments) connected in series with the respiratory gas analyser. High-purity grade nitrogen and oxygen were used for investigating the lower limit of the dynamic range for all channels and the upper limit for the oxygen channel.

For each gas mixture, 3 seconds worth of steady-state $O_2$ and $CO_2$ concentration data points, returned every 10 ms by regression analysis, were recorded and analyzed to construct the data points shown in Fig. 7 D to F. The returned mean fraction ($\mu$) and one standard deviation ($\pm 1\sigma$) are shown as symbol with error bars in the x and y axes representing the uncertainty of the composition of the reference mixture and gas analyser output, respectively.

As previously stated, one aim of the molecular flow sensor is to deliver breath-by-breath measurements of the $O_2$ and $CO_2$ exchange, *i.e.* the volumes of oxygen uptake and carbon dioxide removed within each breath cycle. The performance of the molecular flow sensor of the invention in accomplishing this task was assessed by conducting an $O_2$ wash-in experiment in a healthy volunteer, from which values of uptake rates for oxygen, carbon dioxide, and nitrogen under both air-breathing and high $O_2$-breathing regimes could be calculated and compared.

The arrangement for the equipment in this experiment was as it follows. The subject, who was seating upright on a chair, was asked to breathe normally through a disposable mouthpiece connected to the respiration inlet port of the respiratory gas analyser measurement head, while recordings were made. A standard heat and moisture exchange (HME) filter (Intersurgical) was located between the mouthpiece and the molecular flow sensor for ensuring bacterial filtration and optimum moisture return. A nose clip 2 was placed on the subject's nose so that nasal rebreathing was prevented. The gas mixture was delivered through a Y-piece connector located at the exhaust side of the respiratory gas analyser at approximately 100 l/min to prevent re-breathing of the expired gas. A respiratory gas humidifier was integrated in the breathing circuit to ensure optimal conditioning of the gas mixture supplied to the subject. The molecular flow sensor contributed a dead space of ~70 ml to the section of the breathing apparatus between the subject's mouth and the Y-piece connector.

Values of the three molecular concentrations (oxygen, carbon dioxide and water), sample temperature, pressure, and respiratory flow, were continuously recorded at an update rate of 100 Hz. After 10 minutes of stable air breathing, the inspired gas was switched to medical-grade $O_2$ (BOC, >99.5% purity). Approximately 10 minutes were allowed for the $N_2$ to be fully washed out from the lungs, after which the gas supply was switched back to laboratory air.

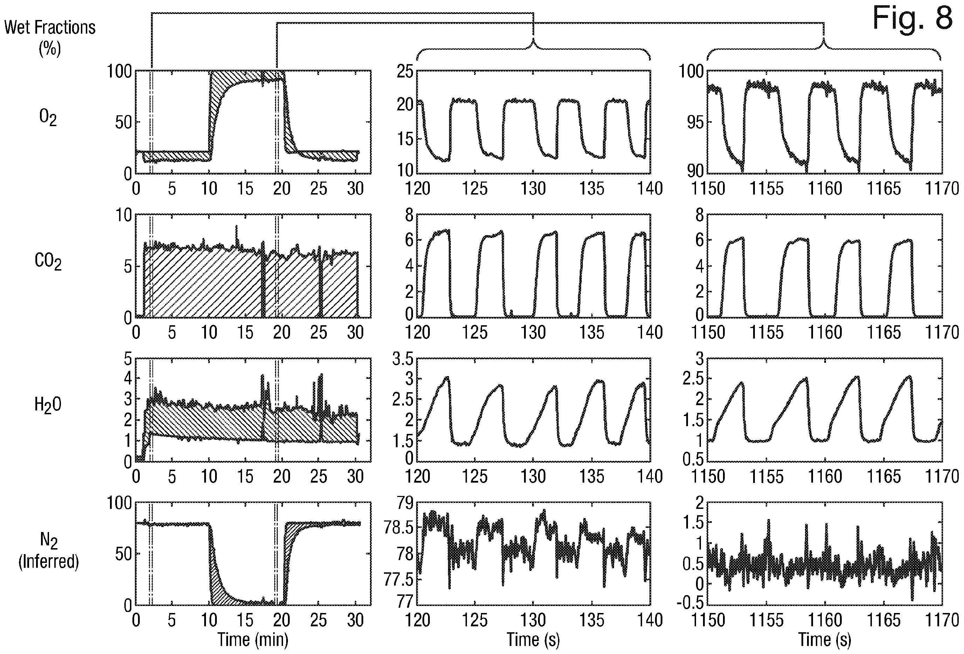

A typical series of the partial pressure profiles for oxygen, carbon dioxide, water vapour, and nitrogen (calculated as balance) retuned by the gas composition analyser during an $O_2$ wash-in experiment are shown in Fig. 8.

**Calculation of gas flows and gas exchange**

The gas analyses enable the instantaneous density and viscosity of the gas mixture to be calculated, and using these and the pressure drop across the flow sensing meshes, the respiratory flow , $\dot{V}$, can be estimated. This together with the gas concentrations measured by the laser spectrometer enables the molecular flow for each measured gas species, *i,* over the 10 ms period, *t,* to be calculated by the product $C_i(t)\dot{V}(t)$.:

The concentration of the balance gas (not analysed) may be calculated by subtracting from the total concentration of gas molecules present (as determined by the instantaneous temperature and pressure) the concentrations of all the gases that have been analysed. The molecular flow of the balance gas over the 10 ms period can then be calculated.

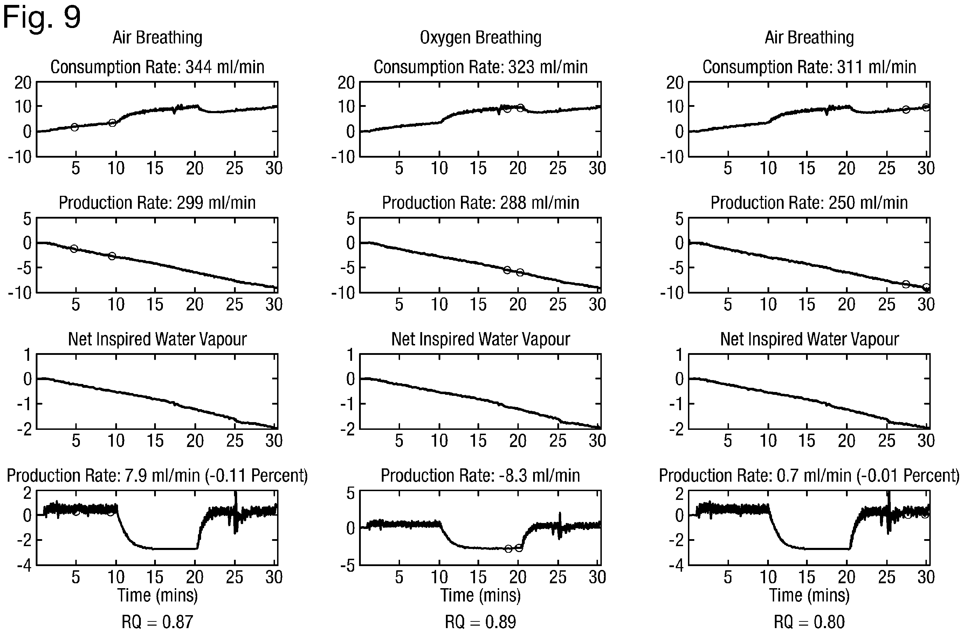

Direct time integration of the product of instantaneous fractional abundance of each gas and the instantaneous flow allowed the net gas volume exchanged to be calculated as function of time. The resulting set of cumulative volume plots are shown in Fig. 9. The general positive trend for $O_2$ indicates that it is consumed, as opposed to the progressive removal of $CO_2$ from the body (negative trend). A closer look shows periodic features representing the end of each inspiration and expiration. For the case of oxygen, each peak corresponds to the end of inspiration (air is breathed in, increasing the oxygen volume within the lungs), and each trough with the end of exhalation (a hypoxic mixture is breathed out). The net volume of nitrogen exchanged was calculated from the total respired volume minus the contributions from oxygen, carbon dioxide, and water vapour. No apparent trend is shown for nitrogen, as expected on the ground that nitrogen does not take part in the gas exchange. Again, small variations in nitrogen content of the lungs, with periodicity of a breath cycle, are noticeable, and can be explained in terms of physiological changes in lung volume and alveolar gas composition, which occur on a breath-by-breath basis.

Table 1 below illustrates a comparison of the performance of the molecular flow sensor of the present invention compared to the specifications published in a consensus statement between the European Respiratory Society and the American Thoracic Society.

**Table 1.**

| Component | Recommended | Molecular Flow Sensor (MFS) |
|---|---|---|
| Volume accuracy | $\pm 3\%$ | $\pm 0.2\%$ |
| Sample Flow | < 20 ml min$^{-1}$ | 0 ml min$^{-1}$ |

(continued)

| Component | Recommended | Molecular Flow Sensor (MFS) |
|---|---|---|
| Gas analyser accuracy | ±1% FSO for $O_2$ | ±0.45% FSO for $O_2$ |
| | ±1% FSO for $CO_2$ | ±0.5% FSO for $CO_2$ |
| | N/A | ±1.7% FSO for $H_2O$ |
| Gas analyser precision | N/A | ±0.18% at 100% $O_2$ |
| | | ±0.39% at 21% $O_2$ |
| | N/A | ±0.20% at 8% $CO_2$ |
| | N/A | ±1.72% at 4.7% $H_2O$ |
| Gas analyser 10-90% rise time | < 100 ms | 10 ms |
| Data sampling frequency | ≥ 100 Hz | 100 Hz |
| Gas/flow analysis synchronisation | 10 ms | 5 ms |

Compared with a mass spectrometer, which is the instrument that probably comes closest to meeting the specifications above, the gas analyser of the invention has no catheter delay (compared with a mass spectrometer of greater than 200 milliseconds), it has a faster response time (10 milliseconds versus 20 milliseconds) and it is capable of measuring the water vapour content. Furthermore, a mass spectrometer is expensive to construct and maintain because of its vacuum system, whereas the components of the present invention are potentially cheap to manufacture in bulk and have no moving parts. The analyser of the invention allows a much more accurate breath-by-breath measurement and this has the significant advantage that breath-to-breath registration of nitrogen exchange at the mouth allows correction of the oxygen consumption and carbon dioxide production for breath-by-breath changes in lung gas stores. It therefore allows the provision of simple, accurate and non-invasive measurements of oxygen consumption in anaesthesia, critical care and cardio-pulmonary exercise testing.

**Calibration of flow meter**

For completeness one method for calibrating the flow meter will be described, though other methods may be used. In this method, which is particularly preferred for its high accuracy, a motorised cylindrical bore pump is used where the stroke volume can be calculated precisely from the diameter of the bore and the stroke length of the piston. Preferably a stroke-volume of similar magnitude to respiratory tidal volume is used (0.5 to 1 litre). It is also useful if the pump has a brief moment of being stationary between inlet and exhaust strokes and so a simple rotary-oscillatory pump is satisfactory. The pump is connected to the measurement head 10 via sealed pipes minimising the compliance and the length and volume of the connections. The pump and respiratory gas analyser are set operating using air or another gas whose exact content is known. The pump is preferably run for several minutes using a variety of pump frequencies and a short period of time (for example 30 seconds) of zero-flow pressure readings are also taken from the differential pressure sensors with the pump not running.

- The pressure-flow relationship in its basic form is:

$$\Delta p = \alpha \mu \dot{V} + \beta \rho \dot{V}^2$$

where $\alpha$ and $\beta$ are constants determined by the fitting, $\dot{V}$ is volumetric flow, $\mu$ is the instantaneous viscosity of the gas, and $\rho$ is the instantaneous density of the gas.
- $\alpha$ and $\beta$ are estimated with use of a non-linear fitting function such as nlinfit.m, a standard function in MATLAB.
- An initial set of parameters are chosen and with the measured differential pressure, the ambient flow is calculated. Using the measured instantaneous airway pressure and airway temperature, the estimated molecular flow is calculated.
- The indices of when the pump was stationary are estimated from applying a peak-finding algorithm to the integral of the pressure-drop.
- The estimated molecular flow is integrated for each pump half-cycle (stroke) using the indices of stationary points as bounds. This provides a vector of the estimated total molecules moved per stroke of length equal to the total number of strokes in the calibration run (typically of the order of 100).
- The objective function for the non-linear fitting function is this vector of estimated total molecules moved per stroke compared to an assumption that a constant number of molecules are moved each stroke cycle and that this number can be calculated from the known and fixed stroke volume.

- The non-linear fitting function returns an optimised set of parameters.

**Claims**

1. A molecular flow sensor for measuring molecular flow along a gas flow path (100), comprising a gas composition analyser integrated within a gas flowmeter, the gas composition analyser being disposed to measure the composition of gas in a measurement space (101) in the flow path (100) of the gas, the gas flowmeter being disposed to provide a signal representing gas flow in the measurement space (101), and a data processor (30) adapted to receive the gas composition measurement and the gas flow signal and to calculate therefrom the flow of each of the individual molecular species analysed by the gas analyser; **characterized in that**:
   the gas flowmeter comprises two flow-sensing mesh screens (102) spaced along the gas flow path (100), a first being located upstream of the measurement space (101) and the second being located downstream of the measurement space (101), and a differential pressure sensor (24) for measuring the pressure difference between the upstream side of the first flow-sensing-mesh screen (102) and the downstream side of the second flow-sensing mesh screen (102).

2. A molecular flow sensor according to claim 1 wherein the two flow-sensing screens (102) are identical to each other.

3. A molecular flow sensor according to claim 1, or 2 wherein the differential pressure sensor (24) is connected to the flow path (100) to sense, via respective pressure-sensing ports (208), the difference in pressure between an upstream side of the first of the two flow-sensing mesh screens (102) and a downstream side of the second of the two flow-sensing mesh screens (102).

4. A molecular flow sensor according to claim 3 further comprising at least two identical flow-conditioning elements (214) respectively and symmetrically positioned upstream and downstream of the upstream and downstream pressure-sensing ports (208).

5. A molecular flow sensor according to claim 4 wherein the at least two identical flow-conditioning elements (214) comprise one or more of flow-conditioning meshes, flow-conditioning metal foam elements, or baffles (216) forming a curved flow path for the gas.

6. A molecular flow sensor according to claim 5 wherein the flow conditioning elements (214) comprise baffles (216), and the baffles form a light trap preventing light from the airway from entering the measurement space (101) and/or the baffles (216) define a helical flow path (100) for the gas.

7. A molecular flow sensor according to any one of the preceding claims wherein each of the pressure sensing ports (208) is connected to a pressure averaging chamber (206) communicating with the gas flow path (100) at a plurality of locations circumferentially spaced around the flow direction and also in communication with the differential pressure sensor (24).

8. A molecular flow sensor according to claim 7 wherein there is a pressure sampling hole (204) at each of said plurality of locations and each of said pressure sampling holes (204) provides communication between the pressure averaging chamber (206) and the respiratory gas flowpath (100).

9. A molecular flow sensor according to claim 7 wherein there is an annular slit extending through said plurality of locations and providing communication between the pressure averaging chamber (206) and the respiratory gas flowpath (100).

10. A molecular flow sensor according to claim 7, 8 or 9 wherein the pressure averaging chamber (206) is an annular channel circumferentially surrounding the respiratory gas flowpath (100).

11. A molecular flow sensor according to any one of the preceding claims wherein the gas composition analyser is an absorption spectrometer for measuring the absorption of light along optical paths through the measurement space (101) and, optionally, the absorption spectrometer comprises an optical cavity spanning the measurement space (101) and formed by mirrors (112) either side of the gas flowpath (100).

12. A molecular flow sensor according to claim 11 wherein the absorption spectrometer comprises a first optical path for oxygen detection and a second optical path for carbon dioxide and water detection.

13. A molecular flow sensor according to any one of the preceding claims further comprising a temperature sensor (15) for sensing the temperature of gas in the flow path (100), the temperature sensor (15) comprising a plurality of thermocouples, at least one positioned axially of the flow path (100) and at least one positioned off axis of the flow path (100).

14. An in-airway molecular flow sensor for measuring respiratory gas flows, comprising a molecular flow sensor according to any one of the preceding claims, and wherein said gas flowmeter is a pneumotachometer disposed in-line in an airway forming said gas flow path (100), said measurement space (101) being in the airway.

15. A pneumotachometer for measuring flow of respiratory gas along an airway, the pneumotachometer comprising: two identical flow-sensing mesh screens (102) spaced along the flowpath (100) of respiratory gas through the airway; a differential pressure sensor (24) connected to the airway to sense, via respective pressure-sensing ports (208), the difference in pressure between an upstream side of the first of the two flow-sensing mesh screens (102) and a downstream side of the second of the two flow-sensing mesh screens; the pneumotachometer further comprising: at least two identical flow-conditioning elements (214) respectively and symmetrically positioned upstream and downstream of the upstream and downstream pressure-sensing ports (208).

## Patentansprüche

1. Sensor für molekulare Strömung zum Messen einer molekularen Strömung entlang eines Gasströmungswegs (100), umfassend einen Gaszusammensetzungsanalysator, der in einen Gasströmungsmesser integriert ist, wobei der Gaszusammensetzungsanalysator so ausgelegt ist, dass er die Zusammensetzung von Gas in einem Messraum (101) in dem Strömungsweg (100) des Gases misst, wobei der Gasströmungsmesser so ausgelegt ist, dass er ein Signal bereitstellt, das eine Gasströmung in dem Messraum (101) darstellt, und einen Datenprozessor (30), der so ausgebildet ist, dass er die Gaszusammensetzungsmessung und das Gasströmungssignal empfängt und anhand dieser die Strömung jeder der einzelnen Molekularspezies berechnet, die vom Gasanalysator analysiert werden, **dadurch gekennzeichnet, dass**:
der Gasströmungsmesser zwei strömungserfassende Maschensiebe (102), die entlang des Gasströmungswegs (100) beabstandet sind, wobei ein erstes dem Messraum (101) vorgeschaltet angeordnet ist und das zweite dem Messraum (101) nachgeschaltet angeordnet ist, und einen Differenzdrucksensor (24) zum Messen des Differenzdrucks zwischen der vorgeschalteten Seite des ersten strömungserfassenden Maschensiebs (102) und der nachgeschalteten Seite des zweiten strömungserfassenden Maschensiebs (102) umfasst.

2. Sensor für molekulare Strömung nach Anspruch 1, wobei die zwei strömungserfassenden Siebe (102) in Bezug aufeinander identisch sind.

3. Sensor für molekulare Strömung nach Anspruch 1 oder 2, wobei der Differenzdrucksensor (24) mit dem Strömungsweg (100) verbunden ist, um die Druckdifferenz zwischen einer vorgeschalteten Seite des ersten der zwei strömungserfassenden Maschensiebe (102) und einer nachgeschalteten Seite des zweiten der zwei strömungserfassenden Maschensiebe (102) über jeweilige druckerfassende Öffnungen (208) zu erfassen.

4. Sensor für molekulare Strömung nach Anspruch 3, die ferner zumindest zwei identische strömungsaufbereitende Elemente (214) umfasst, die jeweils den vorgeschalteten und nachgeschalteten druckerfassenden Öffnungen (208) symmetrisch vorgeschaltet und nachgeschaltet positioniert sind.

5. Sensor für molekulare Strömung nach Anspruch 4, wobei die zumindest zwei identischen strömungsaufbereitenden Elemente (214) eines oder mehrere von strömungsaufbereitenden Gittern, strömungsaufbereitenden Metallschaumelementen oder Umlenkblechen (216) umfassen, die einen gekrümmten Strömungsweg für das Gas bilden.

6. Sensor für molekulare Strömung nach Anspruch 5, wobei die strömungsaufbereitenden Elemente (214) Umlenkbleche (216) umfassen und die Umlenkbleche eine Lichtfalle bilden, um zu verhindern, dass Licht aus den Atemwegen in den Messraum (101) gelangt, und/oder die Umlenkbleche (216) einen helixförmigen Strömungsweg (100) für das Gas definieren.

**7.** Sensor für molekulare Strömung nach einem der vorstehenden Ansprüche, wobei jede der druckerfassenden Öffnungen (208) mit einer Druckmittelungskammer (206) verbunden ist, die mit dem Gasströmungsweg (100) an einer Mehrzahl von Stellen kommuniziert, die um die Strömungsrichtung umfangsmäßig beabstandet sind, und auch mit dem Differenzdrucksensor (24) in Kommunikation steht.

**8.** Sensor für molekulare Strömung nach Anspruch 7, wobei ein Druckprobennahmeloch (204) an jeder der Mehrzahl von Stellen vorgesehen ist und jedes der Druckprobennahmelöcher (204) eine Kommunikation zwischen der Druckmittelungskammer (206) und dem Atemgasströmungsweg (100) bereitstellt.

**9.** Sensor für molekulare Strömung nach Anspruch 7, wobei ein ringförmiger Schlitz vorgesehen ist, der sich durch die Mehrzahl von Stellen erstreckt und eine Kommunikation zwischen der Druckmittelungskammer (206) und dem Atemgasströmungsweg (100) bereitstellt.

**10.** Sensor für molekulare Strömung nach Anspruch 7, 8 oder 9, wobei die Druckmittelungskammer (206) ein ringförmiger Kanal ist, der den Atemgasströmungsweg (100) umfangsmäßig umgibt.

**11.** Sensor für molekulare Strömung nach einem der vorstehenden Ansprüche, wobei der Gaszusammensetzungsanalysator ein Absorptionsspektrometer zum Messen der Absorption von Licht entlang von Strahlengängen durch den Messraum (101) ist und das Absorptionsspektrometer optional einen optischen Resonator umfasst, der den Messraum (101) überspannt und durch Spiegel (112) auf jeder Seite des Gasströmungswegs (100) gebildet ist.

**12.** Sensor für molekulare Strömung nach Anspruch 11, wobei das Absorptionsspektrometer einen ersten Strahlengang zum Sauerstoffnachweis und einen zweiten Strahlengang zum Kohlendioxid- und Wassernachweis umfasst.

**13.** Sensor für molekulare Strömung nach einem der vorstehenden Ansprüche, der ferner einen Temperatursensor (15) zum Erfassen der Temperatur von Gas in dem Strömungsweg (100) umfasst, wobei der Temperatursensor (15) eine Mehrzahl von Thermoelementen umfasst, wobei zumindest eines axial des Strömungswegs (100) positioniert ist und zumindest eines nicht auf der Achse des Strömungswegs (100) positioniert ist.

**14.** Sensor für molekulare Strömung in Atemweg zum Messen von Atemgasströmungen, der einen Sensor für molekulare Strömung nach einem der vorstehenden Ansprüche umfasst, und wobei der Gasströmungsmesser ein Pneumotachometer ist, das in einem Atemweg, der den Gasströmungsweg (100) bildet, linear angeordnet ist, wobei der Messraum (101) in dem Atemweg liegt.

**15.** Pneumotachometer zum Messen einer Atemgasströmung entlang eines Atemwegs, wobei der Pneumotachometer umfasst: zwei identische strömungserfassende Maschensiebe (102), die entlang des Strömungswegs (100) des Atemgases durch den Atemweg beabstandet sind; einen Differenzdrucksensor (24), der mit dem Atemweg verbunden ist, um die Druckdifferenz zwischen einer vorgeschalteten Seite des ersten der zwei strömungserfassenden Maschensiebe (102) und einer nachgeschalteten Seite des zweiten der zwei strömungserfassenden Maschensiebe über jeweilige druckerfassende Öffnungen (208) zu erfassen; wobei das Pneumotachometer ferner umfasst: zumindest zwei identische strömungsaufbereitende Elemente (214), die jeweils den vorgeschalteten und nachgeschalteten druckerfassenden Öffnungen (208) symmetrisch vorgeschaltet und nachgeschaltet positioniert sind.

**Revendications**

**1.** Capteur de débit moléculaire pour mesurer un débit moléculaire le long d'un trajet de débit gazeux (100), comprenant un analyseur de composition gazeuse intégré dans un débitmètre gazeux, l'analyseur de composition gazeuse étant disposé pour mesurer la composition de gaz dans un espace de mesure (101) dans le trajet de débit (100) du gaz, le débitmètre gazeux étant disposé pour fournir un signal représentant le débit gazeux dans l'espace de mesure (101), et un processeur de données (30) adapté pour recevoir la mesure de composition gazeuse et le signal de débit gazeux et pour calculer à partir de ceux-ci le débit de chacune des espèces moléculaires individuelles analysées par l'analyseur gazeux ; **caractérisé en ce que** :
le débitmètre gazeux comprend deux tamis à mailles de détection de débit (102) espacés le long du trajet de débit gazeux (100), un premier étant situé en amont de l'espace de mesure (101) et le deuxième étant situé en aval de l'espace de mesure (101), et un capteur de pression différentielle (24) pour mesurer la différence de pression entre le côté amont du premier tamis à mailles de détection de débit (102) et le côté aval du deuxième tamis à mailles de détection de débit (102).

**2.** Capteur de débit moléculaire selon la revendication 1, dans lequel les deux tamis de détection de débit (102) sont identiques l'un à l'autre.

**3.** Capteur de débit moléculaire selon la revendication 1 ou 2, dans lequel le capteur de pression différentielle (24) est connecté au trajet de débit (100) pour détecter, via des ports de détection de pression respectifs (208), la différence de pression entre un côté amont du premier des deux tamis à mailles de détection de débit (102) et un côté aval du deuxième des deux tamis à mailles de détection de débit (102).

**4.** Capteur de débit moléculaire selon la revendication 3, comprenant en outre au moins deux éléments de conditionnement du débit identiques (214) positionnés respectivement et symétriquement en amont et en aval des ports de détection de pression en amont et en aval (208).

**5.** Capteur de débit moléculaire selon la revendication 4, dans lequel lesdits au moins deux éléments de conditionnement du débit identiques (214) comprennent un ou plusieurs éléments parmi des mailles de conditionnement du débit, des éléments en mousse métallique de conditionnement du débit, ou des déflecteurs (216) formant un trajet de débit incurvé pour le gaz.

**6.** Capteur de débit moléculaire selon la revendication 5, dans lequel les éléments de conditionnement du débit (214) comprennent des déflecteurs (216), et les déflecteurs forment un piège de lumière empêchant la lumière issue de la voie aérienne d'entrer dans l'espace de mesure (101) et/ou les déflecteurs (216) définissent un trajet de débit hélicoïdal (100) pour le gaz.

**7.** Capteur de débit moléculaire selon l'une quelconque des revendications précédentes, dans lequel chacun des ports de détection de pression (208) est connecté à une chambre de moyennage de pression (206) communiquant avec le trajet de débit gazeux (100) à une pluralité d'emplacements espacés circonférentiellement autour de la direction de débit et également en communication avec le capteur de pression différentielle (24).

**8.** Capteur de débit moléculaire selon la revendication 7, dans lequel il y a un trou d'échantillonnage de pression (204) à chaque emplacement de ladite pluralité d'emplacements et chacun desdits trous d'échantillonnage de pression (204) fournit une communication entre la chambre de moyennage de pression (206) et le trajet de débit gazeux respiratoire (100).

**9.** Capteur de débit moléculaire selon la revendication 7, dans lequel il y a une fente annulaire s'étendant au travers de ladite pluralité d'emplacements et fournissant une communication entre la chambre de moyennage de pression (206) et le trajet de débit gazeux respiratoire (100).

**10.** Capteur de débit moléculaire selon la revendication 7, 8 ou 9, dans lequel la chambre de moyennage de pression (206) est un canal annulaire entourant circonférentiellement le trajet de débit gazeux respiratoire (100).

**11.** Capteur de débit moléculaire selon l'une quelconque des revendications précédentes, dans lequel l'analyseur de composition gazeuse est un spectromètre d'absorption pour mesurer l'absorption de lumière le long de trajets optiques au travers de l'espace de mesure (101) et, éventuellement, le spectromètre d'absorption comprend une cavité optique s'étendant sur l'espace de mesure (101) et formée par des miroirs (112) de chaque côté du trajet de débit gazeux (100).

**12.** Capteur de débit moléculaire selon la revendication 11, dans lequel le spectromètre d'absorption comprend un premier trajet optique pour la détection d'oxygène et un deuxième trajet optique pour la détection de dioxyde de carbone et d'eau.

**13.** Capteur de débit moléculaire selon l'une quelconque des revendications précédentes, comprenant en outre un capteur de température (15) pour détecter la température du gaz dans le trajet de débit (100), le capteur de température (15) comprenant une pluralité de thermocouples, au moins un positionné axialement par rapport au trajet de débit (100) et au moins un positionné désaxialement par rapport au trajet de débit (100).

**14.** Capteur de débit moléculaire dans une voie aérienne pour mesurer des débits gazeux respiratoires, comprenant un capteur de débit moléculaire selon l'une quelconque des revendications précédentes, et dans lequel ledit débitmètre gazeux est un pneumotachomètre disposé en ligne dans une voie aérienne formant ledit trajet de débit gazeux (100), ledit espace de mesure (101) étant dans la voie aérienne.

15. Pneumotachomètre pour mesurer un débit de gaz respiratoire le long d'une voie aérienne, le pneumotachomètre comprenant : deux tamis à mailles de détection de débit identiques (102) espacés le long du trajet de débit (100) d'un gaz respiratoire au travers de la voie aérienne ; un capteur de pression différentielle (24) connecté à la voie aérienne pour détecter, via des ports de détection de pression respectifs (208), la différence de pression entre un côté amont du premier des deux tamis à mailles de détection de débit (102) et un côté aval du deuxième des deux tamis à mailles de détection de débit ; le pneumotachomètre comprenant en outre : au moins deux éléments de conditionnement de débit identiques (214) positionnés respectivement et symétriquement en amont et en aval des ports de détection de pression en amont et en aval (208).

# Fig. 1

Breath In/Out

# Fig. 2(A)

# Fig. 2(B)

100 mm

EP 3 314 213 B1

## Fig. 3

# Fig. 4(A)

20, 22

208

100

206

204

200

202

204

# Fig. 4(B)

A

200

208

A

# Fig. 4(C)

Fig. 4(D)

204

208

206

# Fig. 4(D)

206

204

# Fig. 5

# Fig. 6

## Fig. 7(A)

## Fig. 7(B)

## Fig. 7(C)

## Fig. 7(D)

## Fig. 7(E)

## Fig. 7(F)

Fig. 8

EP 3 314 213 B1

# Fig. 9

Air Breathing

Consumption Rate: 344 ml/min

Production Rate: 299 ml/min

Net Inspired Water Vapour

Production Rate: 7.9 ml/min (-0.11 Percent)

Time (mins)

RQ = 0.87

Oxygen Breathing

Consumption Rate: 323 ml/min

Production Rate: 288 ml/min

Net Inspired Water Vapour

Production Rate: -8.3 ml/min

Time (mins)

RQ = 0.89

Air Breathing

Consumption Rate: 311 ml/min

Production Rate: 250 ml/min

Net Inspired Water Vapour

Production Rate: 0.7 ml/min (-0.01 Percent)

Time (mins)

RQ = 0.80

EP 3 314 213 B1

# Fig. 10

Prior Art

Breath In/Out

Fig. 11

16 — 516A

16 —

510A

22B

214 102 100 102 214

22A

28B

20A 12

510B

16 —

16 — 516B

## Fig. 12

Switch 1 { Flow Sensing / Zeroing

Switch 2 { Flow Sensing / Zeroing

Time

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010058150 A1
- US 2009056472 A1
- EP 2404552 A1
- US 5060655 B
- WO 2010058150 A

### Non-patent literature cited in the description

- **C. BLACK ; M. P. W. GROCOTT ; M. SINGER.** Metabolic monitoring in the intensive care unit: $\alpha$ comparison of the Medgraphics Ultima, Deltatrac II, and Douglas bag collection methods. *Br. J. Anaesth.,* 2014, vol. 114, 261-268